(19) Europäisches Patentamt
European Patent Office
Office européen des brevets

(11) **EP 3 021 071 B1**

(12) **EUROPÄISCHE PATENTSCHRIFT**

(45) Veröffentlichungstag und Bekanntmachung des Hinweises auf die Patenterteilung:
**23.09.2020 Patentblatt 2020/39**

(51) Int Cl.:
*G01B 9/02* (2006.01)      *A61B 3/10* (2006.01)

(21) Anmeldenummer: **14405080.4**

(22) Anmeldetag: **12.11.2014**

(54) **Vermessungsverfahren in der Ophthalmologie**

Surveying method for the ophthalmology

Procédé de mesure dans l'ophthalmologie

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.05.2016 Patentblatt 2016/20**

(73) Patentinhaber: **HAAG-STREIT AG**
**CH-3098 Köniz (CH)**

(72) Erfinder:
- **Meznaric, André**
  **3098 Koeniz (CH)**
- **von Waldkirch, Bernhard**
  **3011 Bern (CH)**
- **Schläppi, Christian**
  **3018 Bern (CH)**
- **Zoss, Christian**
  **3123 Belp (CH)**
- **Rindlisbacher, Ernst**
  **3067 Boll (CH)**
- **Breitenstein, Jörg**
  **3052 Zollikofen (CH)**
- **Baltzer, Kaspar**
  **3012 Bern (CH)**
- **Robledo, Lucio**
  **3013 Bern (CH)**
- **Stalder, Peter**
  **4805 Brittnau (CH)**
- **Kiriyanthan, Silja**
  **3097 Liebefeld (CH)**

(74) Vertreter: **Keller & Partner Patentanwälte AG**
**Eigerstrasse 2**
**Postfach**
**3000 Bern 14 (CH)**

(56) Entgegenhaltungen:
**WO-A2-2007/091991     DE-A1- 19 912 428**
**JP-A- 2010 046 216     US-A- 5 988 508**

- **TOMAS TUMA ET AL: "Paper;High-speed multiresolution scanning probe microscopy based on Lissajous scan trajectories;High-speed multiresolution scanning probe microscopy based on Lissajous scan trajectories", NANOTECHNOLOGY, IOP, BRISTOL, GB, Bd. 23, Nr. 18, 20. April 2012 (2012-04-20), Seite 185501, XP020222424, ISSN: 0957-4484, DOI: 10.1088/0957-4484/23/18/185501**
- **HYEON-CHEOL PARK ET AL: "Forward imaging OCT endoscopic catheter based on MEMS lens scanning", OPTICS LETTERS, OPTICAL SOCIETY OF AMERICA, Bd. 37, Nr. 13, 1. Juli 2012 (2012-07-01), Seiten 2673-2675, XP001576879, ISSN: 0146-9592, DOI: 10.1364/OL.37.002673 [gefunden am 2012-06-26]**

**Beschreibung**

**Technisches Gebiet**

Die Erfindung betrifft ein Verfahren zum Erfassen von Messpunkten auf einem Körper, wobei Messpunkte auf einer Fläche des Körpers, mit einem Messstrahl entlang einer Trajektorie erfasst werden.

**Stand der Technik**

In der Augenheilkunde sind diverse Verfahren und Vorrichtungen bekannt, womit das Auge vermessen werden kann. Als nicht-invasive, berührungsfreie und präzise Methode bietet sich dazu zum Beispiel die optische Interferometrie an. Weiter gibt es zahlreiche andere Methoden, z.B. Ultraschall-Biometrie, (Placido-)Hornhauttopographie, Scheimpflug-Kameras, Spaltlampenmikroskope usw. Typischerweise erfolgen solche Untersuchungen am Auge mit einem Interferometer. Als Interferometer werden zum Beispiel OCT (optische Kohärenztomographen) eingesetzt, welche das Auge Punkt für Punkt in lateraler Richtung abtasten und dabei entlang des optischen Strahls Streuchlichtprofile (axiale Profile) des Auges erfassen.

Ryan P. McNabb et al offenbaren im Artikel "Distributed scanning volumetric SCOCT for motion corrected corneal biometry" (01. September 2012, Vol. 3, No. 9 Biomedical Optics Express) ein Scanverfahren, welches unter Verwendung von DSOCT (distributed scanning OCT) topographien eines Auges erfassen kann. Bewegungen des Auges werden dabei mittels SDOCT (spectral domain OCT) ermittelt und in die Messung einbezogen. Das Auge wird entlang einer Trajektorie abgetastet, welche in einer Projektion eine Kreisscheibe abdeckt. Die Trajektorie startet im Randbereich und verläuft entlang einer ersten Geraden durch den Kreismittelpunkt. Anschliessend wird der Messstrahl am gegenüberliegenden Randbereich derart umgelenkt, dass dieser entlang einer, zur ersten Geraden um 9° versetzten zweiten Geraden, durch den Kreismittelpunkt hindurch zurückläuft, um im Randbereich wiederum in derselben Richtung umgelenkt zu werden. Dieses Verfahren wird wiederholt, bis der Anfangspunkt wieder erreicht ist.

In der Publikation vom 01. September 2011, Vol. 2, No. 9, Biomedical Optics Express wird von Karol Karnowski et al. ein Scanverfahren offenbart, bei welchem mit einem Messstrahl entlang rechtwinklig zueinander angeordneten Geraden, d.h. entlang eines Gitternetzes, verfahren wird.

Die EP 1 975 550 A1 offenbart ein optisches System zum Vermessen eines Auges unter Verwendung von OCT. Als Trajektorie werden einerseits eine Spiralenform und anderseits parallel verlaufende Linien offenbart.

Die EP 2 417 903 offenbart weiter konzentrische Ringe, sowie Raster und parallele Linien als Trajektorienformen, welche von einem Messstrahl abgefahren werden.

Die bekannten Verfahren haben den Nachteil, dass die Messungen relativ lange dauern. Aufgrund der relativ langen Messdauer besteht eine erhöhte Gefahr, dass die Messergebnisse durch Augenbewegungen des Patienten beeinflusst werden, womit lediglich ungenaue Messresultate erreicht werden können.

Als wesentliche Anforderungen an die Vermessung, insbesondere bei der Vermessung des Auges, werden einerseits eine geringe Gesamtdauer der Messung sowie eine hohe Auflösung, d.h. eine grosse Abdeckung der zu vermessenden Fläche, gestellt.

**Darstellung der Erfindung**

Aufgabe der Erfindung ist es, ein dem eingangs genannten technischen Gebiet zugehörendes Verfahren zum Erfassen von Messpunkten auf einem Körper zu schaffen, welches bei hoher Auflösung besonders schnell durchführbar ist.

Die Lösung der Aufgabe ist durch die Merkmale des Anspruchs 1 definiert. Gemäss der Erfindung beträgt ein minimaler Krümmungsradius der Trajektorie mindestens 1/7, bevorzugt mindestens 1/5, besonders bevorzugt mindestens 1/3 eines Radius eines Umkreises der Fläche.

Der Erfindung liegt weiter eine Vorrichtung zur Durchführung dieses Verfahrens gemäss Anspruchs 19 zu Grunde.

Die Vorrichtung umfasst dazu vorzugsweise eine der nachfolgend erwähnten Messeinrichtungen, vorzugsweise ein Interferometer, besonders bevorzugt ein OCT-Gerät. Die Messeinrichtung umfasst eine Steuerung, welche eine Führung eines Messstrahls gemäss obigem Verfahren ermöglicht. Solche Steuerungen sind dem Fachmann hinreichend bekannt.

Dem **Messverfahren** liegt prinzipiell eine Längenmessung, insbesondere eine axiale Längenmessung in Z-Richtung (siehe unten), besonders bevorzugt ein axiales Streuprofil (ein sogenannter A-Scan) entlang der Strahlrichtung zugrunde. Das Messverfahren kann aber auch als anderweitige direkte oder indirekte Messung vorliegen. Als indirekte Messung kann eine Laufzeitmessung, insbesondere zum Beispiel mit einem Laser, oder akustisch mittels Echolot vorgesehen sein. Direkt können die Längen konfokal oder interferometrisch bestimmt werden. Als interferometrisches Messverfahren kann zum Beispiel OCT eingesetzt werden. Dem Fachmann sind auch weitere geeignete Messtechniken bekannt.

Nachfolgend werden **X, Y und Z-Koordinaten** als dreidimensionales Orthogonalsystem verwendet. Die Trajektorie wird vorzugsweise entlang einer X,Y-Ebene betrachtet und der Messstrahl weist zumindest in einer Messrichtung, insbesondere in Richtung des Mittelpunkts des Umkreises, die Z-Richtung auf. Der Messstrahl kann aus dieser Lage entweder parallel verfahren, das heisst die Z-Richtung beibehalten, oder aber um einen Winkel in X-Richtung und einen zweiten Winkel in Y-Richtung verschwenkt werden. Weiterhin kann die Lageänderung auch durch eine Kombination dieser Varianten erreicht werden. Sofern nichts anderes erwähnt ist, wird ohne Beschränkung auf eine der Varianten, nachfolgend von der bevorzugten ersteren ausgegangen. Dazu können telezentrische Optiken vorgesehen sein, bei welchen ein Strahlversatz parallel erfolgen kann. Die Längenmessung bezieht sich auf die Z-Achse oder die Richtung des Messstrahls, wobei Änderungen des Brechungsindex entlang der Messachse zu Brechungseffekten führen können, die als axiales Streuprofil erfasst werden. Verfahren zur geometrischen Korrektur und Umrechnungen der gemessenen Längen sind dem Fachmann geläufig.

Die nachfolgend näher beschriebene Trajektorie wird jeweils als **ebene Projektion** in einer X,Y-Ebene verstanden, welche den zu vermessenden Körper schneidet oder einen geringen Abstand zum zu vermessenden Körper aufweist. In der Praxis kann die Trajektorie abhängig vom zu vermessenden Körper von dieser Beschreibung abweichen. Werden zum Beispiel mittels des Messstrahls entlang einer Trajektorie in regelmässigem Abstand Punkte erfasst, so liegen diese Punkte auf dem zu vermessenden Körper nur dann in konstantem Abstand, wenn der Körper eine zur ebenen Projektion parallele Ebene ist. Wird diese Trajektorie aber zum Beispiel auf eine Kalotte projiziert, so liegen zeitlich benachbarte zentrumsnahe Messpunkte näher beieinander als zeitlich benachbarte Messpunkte im Randbereich der Kalotte.

Der zu vermessende **Körper** kann prinzipiell beliebig sein. Je nach Ausbildung des Körpers, d.h. je nach Material und Grösse des Körpers oder je nachdem ob es sich um ein Lebewesen handelt, kann das nachfolgend erläuterte Messverfahren entsprechend gewählt respektive adaptiert und parametrisiert werden. Vorzugsweise wird das Verfahren jedoch zur Vermessung respektive zum Bestimmen von Messpunkten auf einem menschlichen oder tierischen, besonders bevorzugt an einem menschlichen Auge, welches allenfalls Kontakt- oder Intraokularlinsen aufweisen kann, eingesetzt. Vorzugsweise werden dabei die Grenzschichten des Auges (der Hornhaut, der Augenlinse, der Retina) vermessen. Diese Grenzschichten lassen sich in der Regel im zentralen Bereich näherungsweise durch Kugelsegmente beschreiben. Die Schnittpunkte des Messstrahls mit einer Grenzschicht liegen also näherungsweise auf einer Kugelkalotte. Dasselbe gilt bei der Vermessung von künstlichen Körpern mit näherungsweise sphärischen Grenzflächen, wie Kontaktlinsen oder Intraokularlinsen. Der zu vermessende Körper weist damit bevorzugt näherungsweise die Form eines Kugelsegments auf und die Trajektorie verläuft in der Praxis vorzugsweise auf einer Kugelkalotte - die Trajektorien werden jedoch jeweils als X,Y-Projektionen in der Ebene betrachtet (siehe unten).

Bei der Anwendung des Verfahrens an einem Auge handelt es sich bei der **gekrümmten Fläche** vorzugsweise um einen Oberflächenbereich, insbesondere die Hornhaut, oder um einen Bereich einer tieferen Schicht des Auges. Die gekrümmte Fläche kann aber auch durch eine eingesetzte Kontaktlinse oder Interokularlinse gegeben sein.

Unter einem **axialen Längenprofil** oder A-Scan ist ein Profil des Körpers entlang der z-Achse respektive der Strahlrichtung zu verstehen. Unter einem B-Scan wird ein axiales Profil entlang eines geraden Schnitts durch den Körper verstanden, welches sich aus einzelnen Messpunkten und Abständen zusammensetzt. Das axiale Längenprofil kann zu einem dreidimensionalen Modell des Körpers oder einem Schnitt durch den Körper, insbesondere des Auges, zusammengefügt werden. Die Daten können jedoch auch anderweitig eingesetzt werden, zum Beispiel zur Simulation eines Strahlengangs, einer Korrekturlinse oder dergleichen.

Der **Abdeckungsgrad** ist derjenige Anteil der zu vermessenden Fläche, in welchem der Abstand von jedem beliebigen Punkt zum nächstgelegenen Messpunkt einen kritischen Wert nicht überschreitet. Für Messungen der Topographie des Auges wird üblicherweise ein Abdeckungsgrad von 100% gefordert, wobei die zu vermessende Fläche mindestens einen Durchmesser von 7.5mm aufweisen und der kritische Abstand 0.5mm betragen sollte. Je nach Anforderung, zum Beispiel bei der Realisierung kürzerer Messzeiten, können jedoch auch geringere Abdeckungsgrade oder kleinere Messflächen verwendet werden. Die Flächen und Abstände beziehen sich auf die x-y-Ebene in welcher die Trajektorie definiert wird.

Der **Krümmungsradius** der Trajektorie wird vorliegend anhand der ebenen Projektion der Trajektorie in der X, Y - Ebene bestimmt. Der Krümmungsradius r ist bei parametrisierter Kurve f(t) = (x(t), y(t)) wie folgt definiert:

$$r = \left| \frac{(x'(t)^2 + y'(t)^2)^{\frac{3}{2}}}{x'(t) \cdot y''(t) - x''(t) \cdot y'(t)} \right|$$

Die **Trajektorie** ist nachfolgend in Bezug auf die X,Y-Ebene als zweidimensionale Kurve definiert, sofern nichts anderes erwähnt ist. Dem Fachmann ist jedoch klar, dass die Trajektorie in der Anwendung als Projektion auf einen Körper typischerweise eine dreidimensionale Raumkurve darstellt, welche gegenüber der Trajektorie verzerrt ist. Da diese Raumkurve aber sowohl von der Trajektorienform als auch von der Körperform abhängt und damit in grossen Bereichen variieren kann, wird nicht weiter darauf eingegangen. Die Trajektorie muss indes nicht zwingend mathematisch exakt einer Funktion oder Definition folgen. Vorzugsweise weist die Trajektorie eine Form auf, welche zumindest für die einzelnen Messpunkte auf der respektive einer der obig beschriebenen Trajektorie liegt. In diesem Fall werden die Funktionen respektive die Formen schliesslich erst durch eine Interpolation der Messpunkte definiert. In der Praxis können die Messpunkte aber auch geringfügig von der Trajektorie abweichen. Die die mittlere Abweichung von der Trajektorie kann je nach Anzahl Messpunkte und Grösse des Objekts zum Beispiel weniger als 5%, vorzugsweise weniger als 1% des Durchmessers des Umkreises der zu vermessenden Fläche betragen.

Durch die **Wahl des minimalen Krümmungsradius** der Trajektorie von mindestens 1/7, bevorzugt mindestens 1/5, besonders bevorzugt mindestens 1/3 eines Radius eines Umkreises der Fläche über den gesamten Trajektorienverlauf kann der Messstrahl besonders schnell verfahren werden, ohne Teile der Vorrichtung, insbesondere eine Strahlablenkeinheit beziehungsweise einen Scanner grossen Beschleunigungen auszusetzen. Bei den bekannten Trajektorien besteht üblicherweise das Problem, dass schnelle Richtungswechsel vollzogen werden müssen. Schnelle Richtungswechsel können jedoch nicht mit beliebiger Geschwindigkeit vollzogen werden. Die Verzögerung und die anschliessende Beschleunigung der Bewegung des Messstrahls verursachen zeitliche Verzögerungen, welche das Messverfahren als ganzes verlangsamen. Die zeitliche Verzögerung wiederum hat zur Folge, dass die Messergebnisse durch Bewegungen des Patienten wesentlich gestört werden können. Je länger eine solche Messung dauert, desto grösser ist auch die Wahrscheinlichkeit, dass der Patient blinzelt und das Messverfahren unter Umständen sogar abgebrochen und neu gestartet werden muss.

Die neuartige Trajektorie mit den relativ grossen Krümmungsradien in Bezug auf die zu vermessende Fläche ermöglicht nun ein Messverfahren durchzuführen, welches relativ zu der Anzahl Messpunkte schnell durchführbar ist, so dass Bewegungen des Körpers respektive des Auges das Messergebnis weniger verfälschen können. Der Krümmungsradius ist aber auch hinreichend klein gewählt, damit die zu vermessende Fläche bei relativ geringer Bahnlänge der Trajektorie hinreichend abgedeckt werden kann, so dass die Trajektorie einen hinreichend grossen Abdeckungsgrad aufweisen kann.

Bei der Vermessung von Kalotten kann das Problem bestehen, dass bei regelmässigen Messpunktverteilungen in der ebenen Projektion die Abstände zur Kalotte entlang der Trajektorie zentrumsnah geringere Änderungen erfahren als im Randbereich. Anders ausgedrückt sind bei konstanten Messpunktabständen in der ebenen Projektion die Abstände auf der Kalotte nicht mehr konstant, sondern nehmen vom Zentrum der Kalotte aus in radialer Richtung zu. Grosse Abstandsänderungen entlang der Strahlrichtung (Z-Achse) zwischen sequentiell erfassten Messpunkten führen jedoch insbesondere bei interferometrischen Messungen zu Einbussen im Signalkontrast (S. Yun et al., Opt. Expr. 12(13), 2977 (2004)). Es ist daher von besonderem Vorteil, wenn bei der Vermessung einer Kalotte, z.B. bei einer Vermessung eines Auges, der Randbereich der ebenen Projektion der Messpunktverteilung entlang der Trajektorie eine höhere Messpunktdichte aufweist, als ein zentrumsnaher Bereich. Damit wird erreicht, dass die Abstandsänderungen entlang der Trajektorie hinreichend gering und somit der Signalkontrast hinreichend hoch bleibt.

Vorzugsweise weist deshalb die Trajektorie im Randbereich der zu vermessenden Fläche einen grossen Krümmungsradius auf. Insbesondere bei der Vermessung von Kugelkalotten ergibt sich damit der Vorteil, dass sich entlang der Trajektorie im Randbereich geringere Abstandsänderungen ergeben, so dass die Bereiche der Kalotte mit der grössten Steigung (d.h. der Randbereich der Kugelkalotte) exakter vermessen werden können.

In Varianten kann der Bereich der Trajektorie mit dem maximalen Krümmungsradius auch zentrumsnah, im Zentrum selbst oder in einem Zwischenbereich zwischen Randbereich und Zentrum liegen. Die optimale Trajektorie richtet sich im Allgemeinen nach den Anforderungen an die Messauflösung, so kann unter Umständen die exakte Form des äusseren Bereichs der Kalotten von untergeordneter Bedeutung sein, womit die grossen Krümmungsradien eher zentrumsnah gewählt werden können.

Vorzugsweise verläuft die Trajektorie zu mindestens 90%, vorzugsweise zu mindestens 95%, besonders bevorzugt vollständig innerhalb des Umkreises. Damit kann die Trajektorie weitgehend vollständig für die Erfassung der Messpunkte eingesetzt werden, womit die Gesamtzeitdauer der Messung verringert werden kann.

In Varianten kann die Trajektorie auch zu grösseren Anteilen der Bahnlänge ausserhalb der zu vermessenden Fläche liegen. Zum Beispiel kann bloss mindestens 80%, 70% oder weniger der Bahnlänge auf die zu vermessende Fläche entfallen. Je nach Grösse der zu vermessenden Fläche kann die ausserhalb der Fläche liegende Bahnlänge ebenfalls variieren. Unter Umständen kann es für besonders kleine Flächen von Vorteil sein, wenn ein relativ grosser Anteil der Bahnlänge ausserhalb der Fläche liegt, zum Beispiel zu mindestens 50% oder mindestens 75%.

Die Fläche richtet sich dabei auf die grösste für die Bestimmung der Messpunkte in Betracht zu ziehende Fläche. Unter Umständen können verschiedene Teilbereiche der Fläche ausgewertet werden, womit die Fläche als Vereinigung der Teilbereiche anzusehen ist.

Vorzugsweise weist die Trajektorie, insbesondere bei mehr als 50%, vorzugsweise bei mehr als 75%, besonders bevorzugt über eine gesamte Bahnlänge der Trajektorie einen maximalen Krümmungsradius auf, welcher kleiner ist als der Radius des Umkreises der Fläche. Damit kann gesamthaft eine Trajektorie gebildet werden, welche in der Summe relativ grosse Krümmungsradien aufweist und somit von einem Messstrahl schnell durchlaufen werden kann.

In Varianten kann die Trajektorie auch Bereiche mit grösseren Krümmungsradien als der Umkreis der Fläche aufweisen. Insbesondere kann die Trajektorie auch gerade Abschnitte mit einem Krümmungsradius von Unendlich aufweisen.

Ein maximaler Krümmungsradius, welcher den Umkreisradius übersteigt hat zur Folge, dass bei innerhalb der Fläche liegender Trajektorie somit auch relativ kleine Krümmungsradien vorgesehen sein müssen - sofern eine hinreichende Abdeckung erreicht werden soll, müssten damit Krümmungsradien kleiner als 50% des Umkreisradius vorgesehen sein, was sich wiederum negativ auf die Messzeit auswirkt.

Bevorzugt ist der maximale Krümmungsradius kleiner als 99%, vorzugsweise kleiner als 95%, insbesondere kleiner als 90% des Radius des Umkreises. Dadurch, dass die gesamte Trajektorie einen kleineren Krümmungsradius als der Umkreis umfasst, kann eine besonders gleichmässige Trajektorie erreicht werden, welche ohne grössere Änderungen in der Krümmung auskommt und somit für einen Messstrahl besonders schnell durchlaufbar ist.

In Varianten kann, wie obig erwähnt, der maximale Krümmungsradius den Umkreisradius erreichen oder übertreffen.

Vorzugsweise nimmt eine Krümmung der Trajektorie hin zu einem Mittelpunkt des Umkreises monoton, insbesondere streng monoton zu. Dadurch, dass im Randbereich der zu vermessenden Fläche die Trajektorie eine geringere Krümmung aufweist als im zentrumsnahen Bereich, wird eine grössere Messpunktdichte erreicht. Dies ist insbesondere bei kalottenförmigen Körpern, wie zum Beispiel bei einem Auge von Vorteil, da damit die Messpunktdichte auf der Kalotte auch im Randbereich hinreichend hoch gehalten werden kann.

In Varianten kann der Krümmungsradius auch hin zum Mittelpunk des Umkreises zunehmen, insbesondere wenn zum Beispiel aufgrund der Form des Körpers der zentrumsnahe Bereich exakter vermessen werden soll.

Bevorzugt weist die Trajektorie einen Schnittpunkt auf, welcher in zeitlichem Abstand mindestens zweimal erfasst wird. Damit kann eine Bewegung des zu vermessenden Körpers detektiert werden. Die Bewegung des Körpers kann damit aus den Messergebnissen gefiltert werden, so dass präzisere Messdaten erreicht werden können.

In Varianten kann die Trajektorie auch lediglich doppelt durchlaufene Punkte aufweisen.

Bevorzugt weist die Trajektorie mindestens zwei voneinander beabstandete Schnittpunkte auf, wobei insbesondere ein Schnittpunkt in zeitlichem Abstand mehr als zweimal erfasst wird. Durch die beiden beabstandeten Schnittpunkte kann die Detektion der Bewegung des Körpers verbessert werden, da damit nicht nur Betrag sondern auch Vorzeichen der Bewegung erkannt werden kann. Ebenso kann die Bewegungsdetektion optimiert werden, indem ein Schnittpunkt mehr als zweimal durchlaufen wird, da damit zeitliche Änderungen der Bewegung detektiert werden können. Generell können auch mehr als zwei zueinander beabstandete Schnittpunkte vorliegen, womit die Bewegungsdetektion weiter optimiert werden kann; insbesondere können somit Bewegungen entlang unterschiedlicher Richtungen detektiert werden.

In Varianten kann auf die mehreren Schnittpunkte respektive die mehr als zweimal durchlaufenen Schnittpunkte auch verzichtet werden.

Vorzugsweise weisen die mindestens zwei Schnittpunkte in der ebenen Projektion einen Schnittwinkel auf welcher grösser als 90° ist.

In Varianten können die Schnittwinkel auch ausschliesslich 90° betragen.

Bevorzugt folgt der Messstrahl einer Trajektorie, welche mehr als zwei Schnittpunkte aufweist, wobei von k*n Schnittpunkten jeweils n Schnittpunkte auf jeweils einem von k konzentrischen Ringen liegen. Besonders bevorzugt weist die Trajektorie mehr als vier Schnittpunkte auf, da bei zwei Schnittpunkten die triviale Lösung mit zwei konzentrischen Ringen oder ein Ring mit den beiden Schnittpunkten möglich ist.

Sofern nichts anderes erwähnt ist, wird beim Begriff Schnittpunkt jeweils von einem einfachen Schnittpunkt ausgegangen, welcher genau zweimal vom Messstrahl durchlaufen wird. Die Trajektorie kann einen Schnittpunkt im Zentrum des Umkreises aufweisen, so dass sich gesamthaft k*n + 1 Schnittpunkte ergeben würden. Vorliegend wird jedoch der Mittelpunkt des Umkreises nicht als einer der konzentrischen Ringe betrachtet, insbesondere auch da dieser in der Regel nicht als einfacher Schnittpunkt vorliegt.

In einem ersten Fall weist die Trajektorie neben einem zentralen Schnittpunkt genau vier Schnittpunkte auf einem Ring oder je zwei Schnittpunkte auf zwei konzentrischen Ringen auf (wie obig erwähnt ist dabei ein allfällig vorhandener Schnittpunkt im Umkreismittelpunkt nicht mitgerechnet).

In einem weiteren Beispiel weist die Trajektorie zum Beispiel 40 Schnittpunkte auf 5 konzentrischen Ringen auf, so dass auf jedem Ring 8 Schnittpunkte zu liegen kommen.

Dem Fachmann ist klar, dass jede beliebige Anzahl von Schnittpunkten gemäss der Primfaktorzerlegung in zwei Faktorgruppen aufgeteilt und einerseits den n Schnittpunkten pro Ring und andererseits den k Ringen zugeordnet werden kann. Die 40 Schnittpunkte können damit in 2 Ringe mit je 20 Schnittpunkten, 4 Ringe mit je 10 Schnittpunkten, 8 Ringe mit je 5 Schnittpunkten, sowie Schnittpunkten und Ringen mutatis mutandis vertauscht aufgeteilt werden.

In Varianten können die Schnittpunkte der Trajektorie auch anderweitig angeordnet werden. So können zum Beispiel auf alternierenden Ringen jeweils n respektive m Schnittpunkte liegen. Dabei kann zum Beispiel n ein Vielfaches von m sein, wobei ein Schnittpunkt n mehr als zweimal durchlaufen wird.

Bevorzugt verringert sich zwischen den drei konzentrischen Ringen mit den grössten Radien, ein Abstand zwischen zwei benachbarten konzentrischen Ringen mit zunehmendem Radius. Damit rücken die Ringe in Richtung des Randbereichs näher zusammen, womit auch die Schnittpunkte näher beieinander liegen. So kann eine Messpunktdichte im Randbereich erhöht werden, um die Messgenauigkeit insbesondere bei sphärischen Objekten, wie zum Beispiel einem Auge, zu verbessern. Gleichzeitig kann die Bewegung des Auges exakter detektiert werden, da im Randbereich des Auges bei lateraler Abstandsänderung in der X, Y - Ebene sich auch ein Abstand in der Z-Richtung stärker verändert und damit zur Detektion der Augenlage verwendet werden kann.

In Varianten können die Ringe auch konstante Abstände aufweisen oder die Abstände in Richtung des Umkreiszentrums abnehmen.

Vorzugsweise liegen Schnittpunkte zwischen der Trajektorie und den alternierenden konzentrischen Ringen jeweils auf einer radial orientierten Geraden. Damit können die Schnittpunkte optimal über die zu vermessende Fläche verteilt werden, insbesondere wenn die Ringe untereinander einen kleineren Abstand aufweisen, als zwei benachbarte Schnittpunkte auf einem einzelnen Ring. Bei jeweils konstanter Anzahl von n Schnittpunkten pro Ring ergibt sich dabei jeweils eine Versetzung des benachbarten Rings in Bezug auf die Schnittpunkte um einen Winkel von 360/2n Grad.

In Varianten können die Ringe auch um einen Winkel von 360/k*n Grad mit k>2 versetzt sein. Weiter kann k < 2, insbesondere k=1 sein, so dass im letzteren Fall eine radial orientierte Gerade, welche einen Schnittpunkt erfasst auf jedem Ring einen Schnittpunkt erfasst. Schliesslich können die Schnittpunkte auf den Ringen auch einer anderen Ordnung folgen oder chaotisch angeordnet sein.

Vorzugsweise verfährt der Messstrahl entlang der Projektion der Trajektorie mit konstanter Winkelgeschwindigkeit. Damit wird eine besonders einfach parametrisierbare und analysierbare Trajektorie erreicht. Zudem ist diese einfach zu kalibrieren.

In Varianten kann die Trajektorie auch mit nicht konstanter Winkelgeschwindigkeit, sondern zum Beispiel mit konstanter Bahngeschwindigkeit oder sowohl nicht konstanter Bahn- als auch Winkelgeschwindigkeit mit dem Messstrahl abgefahren werden.

Bevorzugt erfolgt die Erfassung der Messpunkte mittels spectral domain OCT (SD-OCT) oder swept source OCT (SS-OCT), vorzugsweise in zeitlich konstanter Frequenz. Verfahren unter Verwendung von OCT haben sich in der Augenheilkunde etabliert, da diese bei streuenden Objekten wie einem Auge eingesetzt werden können und insbesondere eine relativ hohe Eindringtiefe bei gleichzeitiger hoher axialer Auflösung aufweisen.

Bei SD-OCT werden unterschiedliche optische Frequenzen eingesetzt. Das Licht wird dispergiert und mittels CCD oder CMOS-Sensor analysiert. Damit kann die volle Messtiefe mit einer einzigen Messung erreicht werden. Bei SS-OCT wird hingegen die optische Frequenz periodisch durchgestimmt und das Interferenzsignal zeitaufgelöst gemessen. Diese Techniken sind dem Fachmann hinreichend bekannt.

In Varianten können auch andere Techniken zur punktuellen Längenmessung oder zur axialen Profilmessung (Erfassung von A-Scans) eingesetzt werden (siehe Interferometer), zum Beispiel eine Laufzeitmessung mittels Laser oder dergleichen.

Vorzugsweise ist die Trajektorie durch Schleifen gegeben, wobei sich benachbarte Schleifen überschneiden und wobei insbesondere jeweils zwei benachbarte Schleifen einen Schnittpunkt aufweisen, welcher auf einem, vorzugsweise zu einem Umkreis der zu vermessenden Fläche, konzentrischen Kreis liegt. Dieser konzentrische Kreis weist bevorzugt einen Radius auf, welcher kleiner ist als der Umkreisradius. Die Schleifen weisen vorzugsweise sämtliche einen gemeinsamen Schnittpunkt auf, welcher bevorzugt im Zentrum des Umkreises liegt. Diese gemeinsamen Schnittpunkte können auch auf einem beliebig kleinen konzentrischen Kreis liegen, wobei die Abstände der Messpunkte auf diesem Kreis kleiner als zum Beispiel 0.5 mm, vorzugsweise kleiner als 0.1 mm, besonders bevorzugt kleiner als 0.01 mm sind. Besonders bevorzugt weist dieser konzentrische Ring einen Durchmesser von weniger als 0.5 mm, vorzugsweise weniger als 0.1 mm, besonders bevorzugt weniger als 0.01 mm auf. Damit kann in einfacher Weise eine hohe Auflösung des zentralen Bereichs des Körpers erreicht werden. Dies kann insbesondere in der Augenheilkunde beim Bestimmen einer Oberflächenform einer Schicht des Auges, von Vorteil sein.

Die Schleifen sind vorzugsweise derart ausgebildet, dass drei aufeinanderfolgende Messpunkte auf der Schleife nicht auf einer Geraden liegen. Je nach Messpunktdichte kann das Kriterium prinzipiell auch auf vier oder mehr aufeinander folgende Messpunkte, welche nicht auf einer Geraden liegen, erweitert werden.

In Varianten kann die Trajektorie auch nicht oder nicht ausschliesslich durch Schleifen gegeben sein, so dass drei aufeinanderfolgende Messpunkte auf einer Geraden liegen können. Falls das Kriterium auf vier, fünf oder mehr Punkte ausgeweitet ist, so könnte in Varianten entsprechend auch ein Trajektorienabschnitt vier, fünf oder mehr aufeinander folgende Messpunkte auf einer geraden liegen. Benachbarte Schleifen müssen sich nicht zwingend überschneiden, respektive die Schnittpunkte müssen nicht zwingend auf einem Kreisring liegen.

Vorzugsweise ist die Trajektorie durch zwei Frequenzen und einen Radius, insbesondere durch genau zwei Frequenzen definiert. Damit wird das Messverfahren einfach kalibrierbar. An den Scanner können geringe Anforderungen an den Frequenzgang gestellt werden.

In Varianten können auch mehr als zwei Frequenzen respektive mehr als ein Radius zur Definition der Trajektorie verwendet werden. Die Trajektorie kann auch polygonal oder anderweitig definiert sein.

Bevorzugt folgt der Messstrahl einer Kurve mit den Koordinaten:

$$x(t) = r_0 * \sin(\omega_B t) * \cos(\omega_T t)$$

$$y(t) = r_0 * \sin(\omega_B t) * \sin(\omega_T t)$$

Dabei sind:

$r_0$:         Radius des Umkreises des Scanmusters

$\omega_B$, $\omega_T$:         Winkelgeschwindigkeit

wobei $r_0$ der Radius des Umkreises der zu vermessenden Fläche ist. Diese Funktion stellt eine besonders einfach zu kalibrierende Trajektorie dar, welche durch genau zwei Frequenzen ($\omega_B$ und $\omega_T$) und einen Radius ($r_0$) definiert ist. Ein weiterer Vorteil dieser Trajektorie liegt darin, dass die Schrittweite in radialer Richtung nach aussen hin abnimmt und damit der Signalkontrast zunimmt. Dies ist insbesondere bei der Vermessung eines Auges von Vorteil, da in radialer Richtung vom Zentrum des Auges her die Steigung der Oberfläche des Auges zunimmt.

In einer besonders bevorzugten Ausführung betragen $\omega_B = 4 * 2\pi/t_{pattern}$ und $\omega_T = 7 * 2\pi/t_{pattern}$, wobei $t_{pattern}$ die Dauer eines Messdurchgangs bezeichnet. Je nach Grösse des zu Vermessenden Gebiets bei gleichbleibender Messpunktdichte respektive je nach gewünschter Messpunktdichte bei konstanter Gebietsgrösse können die Frequenzen aber auch anders gewählt werden, zum Beispiel $\omega_B = 4 * 2\pi/t_{pattern}$ und $\omega_T = 5 * 2\pi/t_{pattern}$ für kleinere Gebiete respektive kleinere Messpunktdichte oder $\omega_B = 4 * 2\pi/t_{pattern}$ und $\omega_T = 11 * 2\pi/t_{pattern}$ für grössere Gebiete respektive grössere Messpunktdichte. Dem Fachmann ist klar, dass die Parametrisierung dieser Trajektorie beliebig vorgenommen werden kann, insbesondere können für die Kreisfreqeunzen auch nicht-ganzzahlige Vielfache von $2\pi/t_{pattern}$ gewählt werden. Dem Fachmann ist ebenfalls klar, dass der Messstrahl nicht mathematisch exakt dieser Funktion folgen muss, sondern dass lediglich die Messpunkte auf der Trajektorie zu liegen kommen. Die Messpunkte müssen auch nicht exakt auf der Trajektorie liegen, sondern können bevorzugt lediglich hinreichend nahe an der Trajektorie liegen, zum Beispiel mit einem Abstand von weniger als 5%, vorzugsweise weniger als 1%, besonders bevorzugt weniger als 0.1% des Durchmessers der zu vermessenden Fläche.

In Varianten kann auch eine andere Funktion zur Definition einer Trajektorie vorgesehen sein. Zum Beispiel kann die Trajektorie durch die folgende Koordinatengleichung der Hypotrochoiden gegeben sein:

$$x(t) = (a - b) * cos(\omega * t) + c * cos\left(\left(\frac{a-b}{b}\right) * \omega * t\right);$$

$$y(t) = (a - b) * sin(\omega * t) - c * sin\left(\left(\frac{a-b}{b}\right) * \omega * t\right)$$

Dabei ist der Radius des Umkreises der zu vermessenden Fläche durch a - b + c gegeben.

Die Hypotrochoide wird im vorliegenden Fall durch den Verlauf eines beliebig langen, radial ausgerichteten "Zeigers" an einem inneren Kreis gebildet, wobei der innere Kreis innerhalb eines grösseren Kreises abgerollt wird. Damit bezeichnet a den Radius des äusseren Kreises, b den Radius des inneren Kreises mit dem Zeiger der Länge c. Folglich ist der Radius des Umkreises der zu vermessenden Fläche durch a - b + c gegeben.

In der hypotrochoiden Form kann das Zentrum bei geeigneter Parametrisierung in einem Bereich offen gelassen werden. Dies ist dann der Fall, wenn b + c < a. Der Radius des inneren offenen Kreises (falls vorhanden) ist damit a - (b + c). Damit können viele unterschiedliche Schnittpunkte im Bereich des Umkreiszentrums erreicht werden, womit die Messgenauigkeit im Zentralen Bereich wiederum vergrössert werden kann. Der Radius des inneren Kreises ist vorzugsweise derart gehalten, dass ein Abdeckungskriterium eingehalten ist, d.h. der Radius kann zum Beispiel 0.25mm oder weniger betragen.

Der Radius des zentralen offenen Bereichs ist genau dann Null, wenn a - b = c ist, womit im Zentrum ein mehrfacher Schnittpunkt liegt. Diese Form ist zu bevorzugen, wenn die Kontrolle respektive die Korrektur der Augenbewegung höher gewichtet werden, als eine grössere Auflösung im zentralen Bereich des Auges.

Dem Fachmann sind beliebige weitere Variationen von Trajektorien bekannt, welche ebenfalls ausschliesslich grosse Krümmungsradien aufweisen und sowohl das Zentrum als auch den Randbereich mit hoher Auflösung abdecken.

Vorzugsweise liegt zu jedem Messpunkt auf der Trajektorie ein zweiter Messpunkt auf derselben Trajektorie in einem Abstand von weniger als 25%, vorzugsweise weniger als 16% des Radius des Umkreises. Besonders bevorzugt weist jeder Kreis auf der zu vermessenden Fläche mit einem kleineren Radius als 25%, vorzugsweise mit einem kleineren Radius als 16%, besonders bevorzugt mit einem kleineren Radius als 5% des Umkreises mindestens einen Messpunkt auf. Damit wird eine optimale Messpunktdichte mit hinreichender Auflösung des Körpers erreicht.

In Varianten kann der Abstand zu einem nächsten Messpunkt auch anders beschränkt werden.

Bevorzugt liegt zu jedem Punkt innerhalb des Umkreises der Fläche im Abstand von maximal 0.5mm, bevorzugt maximal 0.25mm, besonders bevorzugt maximal 0.1mm ein Messpunkt auf der Trajektorie. Damit kann insbesondere bei einer Augenvermessung eine optimale Abdeckung der zu vermessenden Fläche mit Messpunkten erreicht werden.

In Varianten kann eine Abdeckung der zu vermessenden Fläche mit Messpunkten auch anderweitig definiert sein. In einer weiteren bevorzugten Varianten kann zum Beispiel vorgesehen sein, dass die Messpunktverteilung auf der zu vermessenden Fläche derart gehalten ist, dass eine innerhalb der zu vermessenden Fläche zu liegen kommende Kreisscheibe mit Radius 0.5mm, bevorzugt 0.25mm, besonders bevorzugt 0.1mm unabhängig von deren Position immer mindestens einen Messpunkt abdeckt.

Die Erfindung betrifft weiter ein Verfahren zur Approximation eines Querschnitts eines Körpers. Die für das Approximationsverfahren benötigten Messpunkte werden bevorzugt unter Verwendung des obig beschriebenen Verfahrens zum Vermessen eines Körpers durchgeführt.

Es ist aber auch denkbar die Messpunkte unter Verwendung eines anderen Verfahrens zu ermitteln, insbesondere durch herkömmliche, dem Fachmann bekannte Messverfahren.

Zur Approximation des Querschnitts des Körpers, vorzugsweise eines Auges, wird dazu im Bereich des Querschnittes des Auges eine Teilmenge von erfassten Messpunkten, welche mindestens einen zum Querschnitt beabstandeten Messpunkt umfassen, zu einer Approximation des Querschnitts verrechnet. Damit können beliebige Querschnitte des Körpers berechnet werden, ohne dass jeweils entsprechende Messpunkte exakt im Querschnitt verfügbar sein müssen.

Bevorzugt ist die Teilmenge der erfassten Messpunkte zweier spiegelsymmetrisch angeordneter Sektoren mit Mittelpunktswinkel kleiner als 90° in einem Umkreis einer zu vermessenden Fläche.

Bei einem Messverfahren mit konstanter Winkelgeschwindigkeit kann damit über den Querschnitt hinweg eine im Wesentlichen konstante Anzahl Messpunkte pro Querschnittslängenabschnitt erreicht werden.

In Varianten kann der Sektor auch nicht durch Geraden begrenzt sein, sondern sich vom Mittelpunkt des Umkreises aus in radialer Richtung nicht linear aufweiten. Damit kann gewichtet werden, dass weiter vom Querschnitt entfernte Messpunkte grössere Unschärfe in der Approximation zur Folge haben, womit in diesen Bereichen mehr Messpunkte einbezogen werden. Dem Fachmann ist klar, dass prinzipiell auch jede anderweitig geformte Fläche zur Approximation eines Querschnitts herangezogen werden kann. Insbesondere kann ein Querschnitt auch nicht linear verlaufen, sondern zum Beispiel V-förmig, als "Kuchenstück" des Objekts ausgebildet sein, so dass das Einzugsgebiet entsprechend zwei Kreissektoren umfasst, welche gegebenenfalls einen Zwischenwinkel aufweisen oder sich berühren, respektive überlappen. Der Querschnitt kann auch anders verlaufen, zum Beispiel Wellenförmig, Polygonal wie zum Beispiel als regelmässiges Vieleck, Zickzack-förmig etc.

Aus der nachfolgenden Detailbeschreibung und der Gesamtheit der Patentansprüche ergeben sich weitere vorteilhafte Ausführungsformen und Merkmalskombinationen der Erfindung.

## Kurze Beschreibung der Zeichnungen

Die zur Erläuterung des Ausführungsbeispiels verwendeten Zeichnungen zeigen:

Fig. 1    ein rasterförmiges Scanmuster gemäss Stand der Technik;

Fig. 2    ein schleifenförmiges Scanmuster gemäss Stand der Technik;

Fig. 3    ein spiralförmiges Scanmuster gemäss Stand der Technik;

Fig. 4    eine erste Ausführungsform eines erfindungsgemässen Scanmusters;

Fig. 5    eine zweite Ausführungsform eines erfindungsgemässen Scanmusters;

Fig. 6    eine dritte Ausführungsform eines erfindungsgemässen Scanmusters;

Fig. 7    eine vierte Ausführungsform eines erfindungsgemässen Scanmusters;

Grundsätzlich sind in den Figuren gleiche Teile mit gleichen Bezugzeichen versehen.

## Wege zur Ausführung der Erfindung

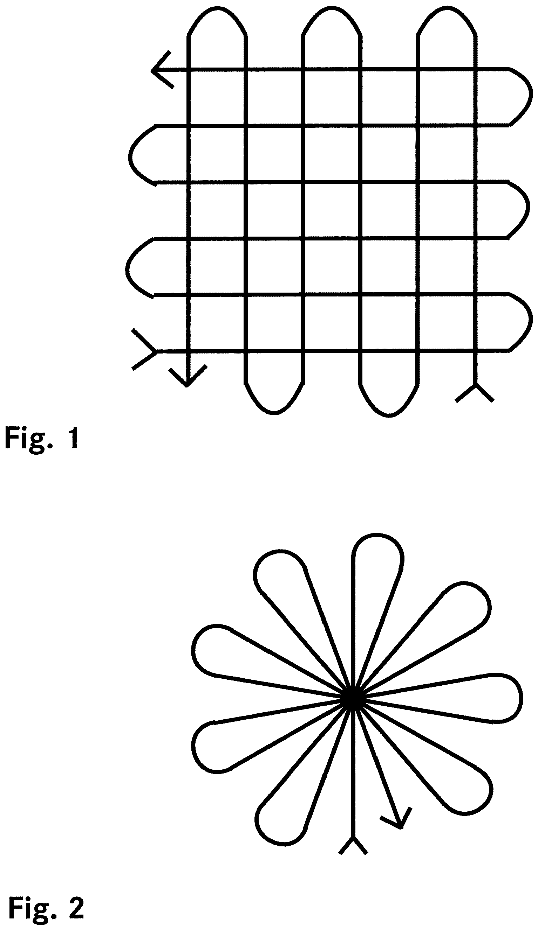

Die **Figur 1** zeigt ein rasterförmiges Scanmuster gemäss dem Stand der Technik. Das Scanmuster ist unter anderem bekannt durch die Röhrenfernseher, bei welchen der Elektronenstrahl zeilenweise den Bildschirm abfährt. Die Figur 1 zeigt zwei gleiche Muster, welche zueinander um 90° versetzte und überlappend sind. Beide Muster werden separat abgefahren. Während die geraden Streckenstücke der Trajektorie relativ schnell abgefahren werden können, ergeben sich jeweils erhebliche Verzögerungen im Randbereich durch die abrupten Richtungsänderungen. Daher kann dieses Scanmuster mittels eines Messstrahls zeitlich nicht hinreichend effizient abgefahren werden. Zudem weist das Scanmuster insbesondere bei einem kreisförmigen respektive kalottenförmigen Körper wie zum Beispiel bei einem Auge im Randbereich dieselbe Messpunktdichte auf, wie im Zentrum. Damit sind dieses Scanmuster respektive diese Trajektorie nicht optimal für das Erfassen von Oberflächen eines Auges ausgebildet.

Die **Figur 2** zeigt ein schleifenförmiges Scanmuster gemäss Stand der Technik. Dieses Scanmuster weist mehrere, vom Zentrum aus gebildete Schlaufen auf. Anders ausgedrückt ist das Scanmuster durch gerade Abschnitte gegeben, welche in regelmässigen Winkelabständen das Zentrum des Umkreises der zu vermessenden Fläche durchlaufen. Jeweils am Ende eines solchen geraden Abschnittes erfolgt über die Schlaufenbildung die Verbindung zum benachbarten Abschnitt, zum Beispiel jeweils im Uhrzeigersinn. Die gebildeten Schlaufenenden weisen einen relativ kleinen Krümmungsradius auf. Je grösser die Winkelabstände der Abschnitte sind, desto grösser ist der Krümmungsradius am Abschnittsende, aber anderseits nimmt zugleich die Gesamtzahl der Abschnitte ab, wodurch auch die Messpunktdichte auf der zu vermessenden Fläche reduziert wird. Damit spielen sich die beiden Faktoren des Krümmungsradius und der Messpunktdichte oder des maximalen Abstands zwischen zwei benachbarten Punkten gegenseitig aus. Ein weiterer Nachteil liegt darin, dass im Zentrum eine sehr grosse Verdichtung von Messpunkten erfolgt, welche nicht in sinnvoller Weise der Auswertung des Oberflächenprofils des Körpers dienlich sind. Gegebenenfalls kann das Scanmuster derart gross gewählt werden, dass ein grosser Teil des Scanmusters ausserhalb der zu vermessenden Fläche liegt. Dies hätte den Vorteil, dass die Krümmungsradien grösser sind und schneller durchlaufen werden können, anderseits würde sich damit die Gesamtweglänge massiv vergrössern, so dass die Messdauer gesamthaft grösser wäre.

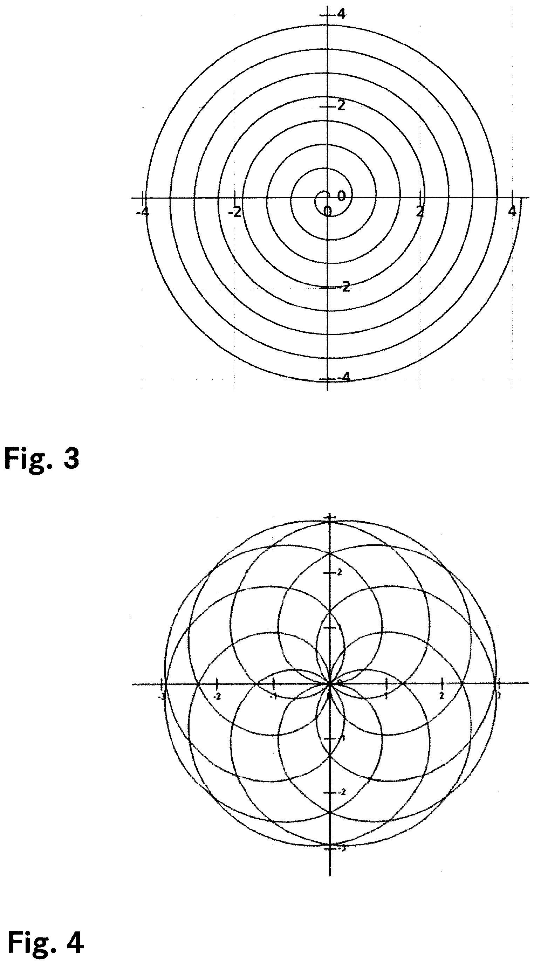

Die **Figur 3** zeigt ein spiralförmiges Scanmuster gemäss Stand der Technik. Während das Spiralmuster im Randbereich hinreichend grosse Krümmungsradien aufweist, welche von einem Messstrahl schnell durchlaufen werden könnten, wird der Krümmungsradius hin zum Zentrum immer kleiner. Da aber der zentrale Bereich insbesondere in der Augenheilkunde von grosser Wichtigkeit ist, ist auch dieses Scanmuster nachteilig, da der zentrale Bereich entweder sehr langsam durchlaufen respektive nur mit geringer Auflösung vermessen werden kann."

In den nachfolgenden **Figuren 4 bis 7** sind vier verschiedene erfindungsgemässe Scanmuster der allgemeinen Form:

$$x(t) = r_0 * \sin(\omega_B t) * \cos(\omega_T t)$$

$$y(t) = r_0 * \sin(\omega_B t) * \sin(\omega_T t)$$

dargestellt. Dabei sind:

$r_0$: Radius des Umkreises des Scanmusters

$\omega_B$:

$\omega_T$:

$$\omega_B = 2\pi \frac{B}{2t_{pattern}},$$

$$\omega_T = 2\pi \frac{T}{t_{pattern}}$$

Für die nachfolgenden Beispiele beträgt die Messdauer $t_{pattern}$ 200ms (Millisekunden). Dem Fachmann ist klar, dass prinzipiell eine kleinstmögliche Messdauer angestrebt ist. Diese ist jedoch einerseits vom eingesetzten Messgerät und anderseits von der Anzahl Messpunkte abhängig.

Die Anzahl Messpunkte ist vorliegend 3200, die Messfrequenz (d.h. die Rate mit welcher Messpunkte erfasst werden) beträgt f = 16 kHz. Angestrebt wird dabei ein Gleichgewicht, bei welchem die Messdauer hinreichend klein und gleichzeitig die Anzahl Messpunkte und damit bei konstanter zu vermessenden Fläche die Auflösung hinreichend gross ist.

Weiterhin ist die Messfrequenz jedoch nur so gross, dass sich pro Messpunkt noch eine ausreichende Signalstärke ergibt, da diese mit zunehmender Messfrequenz abnimmt. Je nach Messsystem kann die Messfrequenz von wenigen kHz bis zu einigen MHz betragen. Als erstrebenwert haben sich Messfrequenzen im Bereich von 10 bis 200 kHz erwiesen.

Je nach eingesetztem Messgerät können die Messdauer und Anzahl Messpunkte auch kleiner respektive höher sein. Je nach Messanordnung kann es von Vorteil sein, wenn die Messdauer verkürzt wird, wobei die kleinere Auflösung in Kauf genommen wird. Anderseits kann auch auf Kosten der Messdauer die Anzahl Messpunkte erhöht werden.

Der Radius der zu vermessenden Fläche ist vorliegend 4mm. Dieser ist jedoch ebenfalls von den spezifischen Anforderungen abhängig und kann prinzipiell beliebig gewählt werden, zum Beispiel 10mm, 3.5mm, 1.5mm sowie sämtliche dazwischenliegenden und aussen liegenden Bereiche.

Die axiale Systemauflösung des Messgeräts ist vorliegend bei ungefähr 4.6μm, aber auch diese kann höher oder niedriger sein.

Dem Fachmann ist klar, dass der Durchmesser, die Anzahl Messpunkte wie auch die Messzeit in anderen Bereichen liegen kann.

Schliesslich ist dem Fachmann auch klar, dass die Trajektorie nicht auf die exakte Einhaltung die generell angegebenen Formeln (zuvor und nachfolgende Formeln) gebildeten Graphen beschränkt ist. Eine Trajektorie oder ein Scanmuster kann auch von der mathematisch exakten Form abweichen. So kann zum Beispiel die mit dem Messstrahl ermittelte Punkteschar lediglich als Interpolation näherungsweise einer solchen Funktion entsprechen.

Die **Figur 4** zeigt eine erste Ausführungsform eines erfindungsgemässen Scanmusters in einer besonders bevorzugten Form mit B = 8 und T = 7. Am Funktionsgraphen ist gut zu erkennen, dass der Krümmungsradius jeweils vom Randbereich aus hin zum Zentrum zunimmt. Zudem liegen jeweils acht Schnittpunkte immer auf einem zum Zentrum des Umkreises konzentrischen Kreis und der Mittelpunkt wird mehrfach durchlaufen. Weiter ist in der Figur ersichtlich, dass sowohl der Randbereich als auch der zentrumsnahe Bereich hoch aufgelöst vermessen werden kann. Das Scanmuster weist 48 einfache Schnittpunkte und einen achtfachen Schnittpunkt im Zentrum auf. Insbesondere mit den Schnittpunkten ausserhalb des Zentrums kann die Augenbewegung detektiert und eliminiert werden. Die hohe Anzahl an Schnittpunkten erlaubt eine Detektion der Augenbewegung in entsprechend hoher Frequenz (Messzeit/Anzahl Schnittpunkte = mittlere Aktualisierungszeit).

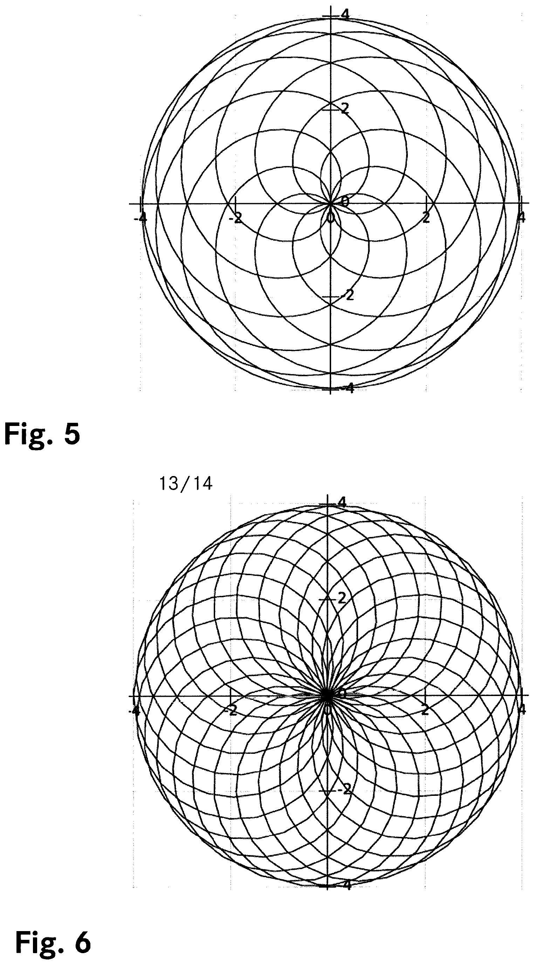

Die **Figur 5** zeigt eine zweite Ausführungsform eines erfindungsgemässen Scanmusters, wobei B = 8 und T = 11 ist. Im Unterschied zum Scanmuster gemäss Figur 4 weist das vorliegende Scanmuster auf derselben Fläche eine grössere Bahnlänge und mehr Schnittpunkte auf. Dies erlaubt eine grössere Auflösung, d.h. kleinere mittlere Distanzen zwischen benachbarten Messpunkten. Die Anzahl einfacher Schnittpunkte beträgt vorliegend 80 bei jeweils 8 Schnittpunkten pro konzentrischen Ring.

Die **Figur 6** zeigt eine dritte Ausführungsform eines erfindungsgemässen Scanmusters, wobei B = 13 und T = 14 ist. Diese Ausführungsform eines Scanmusters weist mit 26 Schnittpunkten pro konzentrischen Ring bei 13 konzentrischen Ringen eine Gesamtzahl von 338 Schnittpunkten auf. Diese Ausführungsform oder auch solche mit noch mehr Schnittpunkten können bei entsprechend schnellen Scannern oder für grösserflächige Objekte Verwendung finden. Mit gegenwärtigen OCT-Scannern dürfte die Messdauer bei einer solchen Trajektorie zu hoch sein.

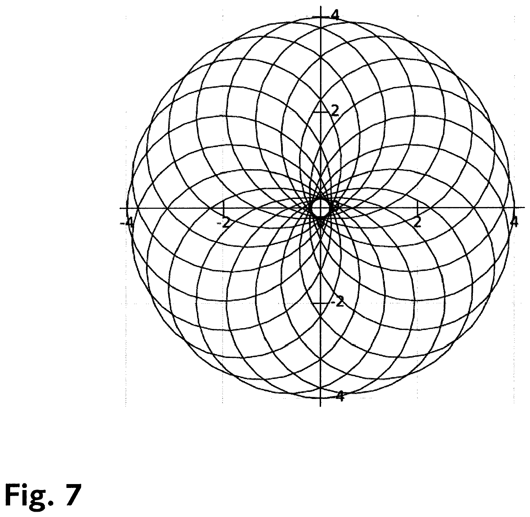

Die **Figur 7** zeigt schliesslich als viertes Beispiel eines hypotrochoiden Scanmusters als mögliche Ausführungsform eines erfindungsgemässen Scanmusters. Das hypotrochoide Scanmuster weist die allgemeine Form:

$$x(t) = (a - b)cos(s) + c * cos\left(\left(\frac{a-b}{b}\right) * s\right);$$

$$y(t) = (a - b)sin(s) - c * sin\left(\left(\frac{a-b}{b}\right) * s\right)$$

auf. Dem Fachmann ist jedoch klar, dass die Trajektorie auch hier nicht auf die exakte Einhaltung der durch die obige Formel gebildeten Graphen beschränkt ist. Eine Trajektorie oder ein Scanmuster kann auch von der mathematisch exakten Form abweichen. So kann zum Beispiel die mit dem Messstrahl ermittelte Punkteschar lediglich als Interpolation näherungsweise einer solchen Funktion entsprechen.

Für die Ermittlung von Messwerten in der Augenheilkunde können die Werte derart gewählt werden, dass wiederum ein Radius von ungefähr 4mm erreicht wird. Als Beispiel ist in der Figur 7 a=2, b=0.1 und c=2.1 gewählt. Mit dieser Parametrisierung ist im Zentrum des Umkreises ein freier Kreis zu erkennen, welcher einen Radius von ungefähr 0.2mm aufweist. Diese Freifläche erfüllt damit das eingangs genannte 0.5mm-Kriterium.

Zusammenfassend ist festzustellen, dass das erfindungsgemässe Verfahren zum Erfassen von Messpunkten besonders schnell durchführbar und damit robust gegenüber Bewegungen des Körpers, insbesondere des Auges ist während gleichzeitig eine hohe Auflösung, insbesondere im Randbereich von kalottenförmigen Körpern, erreichbar ist.

**Patentansprüche**

1. Verfahren zum Erfassen von Messpunkten auf einem Auge, wobei Messpunkte auf einer gekrümmten Fläche des Auges zur interferometrischen Erfassung eines axialen Längenprofils, mit einem Messstrahl entlang einer Trajektorie erfasst werden, **dadurch gekennzeichnet, dass** ein minimaler Krümmungsradius der Trajektorie mindestens 1/7, bevorzugt mindestens 1/5, besonders bevorzugt mindestens 1/3 eines Radius eines Umkreises der Fläche beträgt und wobei die Trajektorie bei mehr als 50%, vorzugsweise bei mehr als 75%, besonders bevorzugt über eine gesamte Bahnlänge der Trajektorie einen maximalen Krümmungsradius aufweist, welcher kleiner ist als der Radius des Umkreises der Fläche.

2. Verfahren nach Anspruch 1, **dadurch gekennzeichnet, dass** die Trajektorie zu mindestens 90%, vorzugsweise zu mindestens 95%, besonders bevorzugt vollständig innerhalb des Umkreises verläuft.

3. Verfahren nach einem der Ansprüche 1 bis 2, **dadurch gekennzeichnet, dass** ein maximaler Krümmungsradius der Trajektorie kleiner als 99%, vorzugsweise kleiner als 95%, insbesondere kleiner als 90% des Radius des Umkreises ist.

4. Verfahren nach einem der Ansprüche 1 bis 3, **dadurch gekennzeichnet, dass** eine Krümmung der Trajektorie hin zu einem Mittelpunkt des Umkreises monoton, insbesondere streng monoton zunimmt.

5. Verfahren nach einem der Ansprüche 1 bis 4, **dadurch gekennzeichnet, dass** die Trajektorie einen Schnittpunkt aufweist, welcher in zeitlichem Abstand mindestens zweimal erfasst wird, womit insbesondere eine Bewegung des Auges detektiert wird.

6. Verfahren nach Anspruch 5, **dadurch gekennzeichnet, dass** die Trajektorie mindestens zwei voneinander beabstandete Schnittpunkte aufweist und wobei insbesondere ein Schnittpunkt in zeitlichem Abstand mehr als zweimal erfasst wird.

7. Verfahren nach Anspruch 6, **dadurch gekennzeichnet, dass** die mindestens zwei Schnittpunkte in der ebenen Projektion einen Schnittwinkel aufweisen welcher grösser als 90° ist.

8. Verfahren nach Anspruch 6 oder 7, **dadurch gekennzeichnet, dass** der Messstrahl einer Trajektorie folgt, welche mehr als zwei Schnittpunkte aufweist, wobei von k*n Schnittpunkten jeweils n Schnittpunkte auf jeweils einem von k konzentrischen Ringen liegen.

9. Verfahren nach Anspruch 8, **dadurch gekennzeichnet, dass** zwischen den drei konzentrischen Ringen mit den grössten Radien, sich ein Abstand zwischen zwei benachbarten konzentrischen Ringen mit zunehmendem Radius verringert.

10. Verfahren nach Anspruch 8 oder 9, **dadurch gekennzeichnet, dass** Schnittpunkte zwischen der Trajektorie und den alternierenden konzentrischen Ringen jeweils auf einer radial orientierten Geraden liegen.

11. Verfahren nach einem der Ansprüche 1 bis 10, **dadurch gekennzeichnet, dass** die Erfassung der Messpunkte mittels spectral domain OCT oder swept source OCT, vorzugsweise in zeitlich konstanter Frequenz erfolgt.

12. Verfahren nach einem der Ansprüche 1 bis 11, **dadurch gekennzeichnet, dass** die Trajektorie durch Schleifen gegeben ist, wobei sich benachbarte Schleifen überschneiden und wobei insbesondere jeweils zwei benachbarte Schleifen einen Schnittpunkt aufweisen, welcher auf einem, zu einem Umkreis der zu vermessenden Fläche, konzentrischen Kreis liegt.

13. Verfahren nach einem der Ansprüche 1 bis 12, **dadurch gekennzeichnet, dass** die Trajektorie durch zwei Frequenzen und einen Radius, insbesondere durch genau zwei Frequenzen definiert ist.

14. Verfahren nach Anspruch 13, **dadurch gekennzeichnet, dass** der Messstrahl einer Kurve mit den Koordinaten $(x(t);y(t)) = (r_0 * \sin(\omega_B t) * \cos(\omega_T t); r_0 * \sin(\omega_B t) * \sin(\omega_T t))$, folgt, wobei $r_0$ der Radius des Umkreises der zu vermessenden Fläche ist.

15. Verfahren nach einem der Ansprüche 1 bis 14, **dadurch gekennzeichnet, dass** zu jedem Messpunkt auf der Trajektorie ein zweiter Messpunkt auf derselben Trajektorie in einem Abstand von weniger als 25%, vorzugsweise weniger als 16% des Radius liegt.

16. Verfahren nach einem der Ansprüche 1 bis 15, **dadurch gekennzeichnet, dass** zu jedem Punkt innerhalb des Umkreises der Fläche im Abstand von maximal 0.5mm, bevorzugt maximal 0.25mm, besonders bevorzugt maximal 0.1mm ein Messpunkt auf der Trajektorie liegt.

17. Verfahren zur Approximation eines Querschnitts eines Auges unter Verwendung von mit einem Verfahren nach einem der Ansprüche 1 bis 16 erfassten Messpunkten, wobei im Bereich des Querschnittes eine Teilmenge von erfassten Messpunkten, welche mindestens einen zum Querschnitt beabstandeten Messpunkt umfassen, zu einer Approximation des Querschnitts verrechnet werden.

18. Verfahren nach Anspruch 17, wobei die Teilmenge der erfassten Messpunkte zweier spiegelsymmetrisch angeordneter Sektoren mit Mittelpunktswinkel kleiner als 90° in einem Umkreis einer zu vermessenden Fläche sind.

19. Vorrichtung, die zur Durchführung eines Verfahrens nach einem der Ansprüche 1 bis 16 konzipiert wurde, mit einer Messeinrichtung eine Steuerung umfassend, welche eine Führung eines Messstrahls gemäss des Verfahrens ermöglicht.

## Claims

1. Method for registering measuring points on an eye, wherein measurement points are registered along a trajectory on a curved surface of the eye for interferometric registering an axial length profile, by way of a measurement beam, **characterized in that** a minimum radius of curvature of the trajectory is at least 1/7, preferably at least 1/5, particularly preferably at least 1/3 of a radius of a circumference of the surface, and wherein the trajectory has a maximum radius of curvature which is less than the radius of the circumference of the surface, in particular over more than 50%, preferably over more than 75%, particularly preferably over a whole path length of the trajectory.

2. Method according to Claim 1, **characterized in that** at least 90%, preferably at least 95%, particularly preferably the whole of the trajectory extends within the circumference.

3. Method according to one of Claims 1 to 2, **characterized in that** the maximum radius of curvature of the trajectory is less than 99%, preferably less than 95%, more particularly less than 90% of the radius of the circumference.

4. Method according to one of Claims 1 to 3, **characterized in that** a curvature of the trajectory toward the center of the circumference increases monotonically, more particularly strictly monotonically.

5. Method according to one of Claims 1 to 4, **characterized in that** the trajectory has a point of intersection which is registered at least twice with a time lag, as a result of which, in particular, a movement of the body is detected.

6. Method according to Claim 5, **characterized in that** the trajectory has at least two spaced apart points of intersection and wherein, in particular, a point of intersection is registered more than twice with a time lag.

7. Method according to Claim 6, **characterized in that** the at least two points of intersection have an angle of intersection which is greater than 90° in the planar projection.

8. Method according to Claim 6 or 7, **characterized in that** the measurement beam follows a trajectory which has more than two points of intersection, wherein, in the case of k*n points of intersection, respectively n points of intersection lie on respectively one of k concentric rings.

9. Method according to Claim 8, **characterized in that** a distance between two adjacent concentric rings with increasing radius is reduced between the three concentric rings with the largest radii.

10. Method according to Claim 8 or 9, **characterized in that** points of intersection between the trajectory and the alternating concentric rings respectively lie on a radially oriented straight line.

11. Method according to one of Claims 1 to 10, **characterized in that** the measurement points are registered by means of spectral domain OCT or swept source OCT, preferably with a time-constant frequency.

12. Method according to one of Claims 1 to 11, **characterized in that** the trajectory is given by loops, wherein adjacent loops intersect and wherein, in particular, two adjacent loops respectively have a point of intersection which lies on a circle concentric with a circumference of the surface to be measured.

13. Method according to one of Claims 1 to 12, **characterized in that** the trajectory is defined by two frequencies and a radius, more particularly by exactly two frequencies.

14. Method according to Claim 13, **characterized in that** the measurement beam follows a curve with the coordinates $(x(t);y(t)) = (r_0 * \sin(\omega_B t) * \cos(\omega_T t); r_0 * \sin(\omega_B t) * \sin(\omega_T t))$, where $r_0$ is the radius of the circumference of the surface to be measured.

15. Method according to one of Claims 1 to 14, **characterized in that**, for each measurement point on the trajectory, there is a second measurement point on the same trajectory at a distance of less than 25%, preferably of less than 16% of the radius.

16. Method according to one of Claims 1 to 15, **characterized in that**, for each point within the circumference of the surface, there is a measurement point on the trajectory at a distance of at most 0.5 mm, preferably at most 0.25 mm, particularly preferably at most 0.1 mm.

17. Method for approximating a cross section of an eye using measurement points registered by a method according to one of Claims 1 to 16, wherein, in the region of the cross section, a subset of registered measurement points, which comprise at least one measurement point at a distance from the cross section, has operations performed thereon in order to approximate the cross section.

18. Method according to Claim 17, wherein the subset of the registered measurement points of two sectors with center-point angles of less than 90° arranged in a mirror symmetric manner is in a circumference of a surface to be measured.

19. Device for carrying out the method according to one of Claims 1 to 16, with a measuring device comprising a controller, wherewith a measuring beam can be guided according to of the method.

**Revendications**

1. Procédé de détection de points de mesure sur un œil, dans lequel des points de mesure situés sur une surface incurvée de l'œil sont détectés au moyen d'un faisceau de mesure le long d'une trajectoire pour la détection interférométrique d'un profil longitudinal axial, **caractérisé en ce qu'**un rayon de courbure minimal de la trajectoire est d'au moins 1/7, de préférence d'au moins 1/5, plus préférablement d'au moins 1/3 d'un rayon d'une circonférence de la surface et dans lequel la trajectoire présente un rayon de courbure maximal, sur plus de 50 %, de préférence sur plus de 75 %, et de manière particulièrement préférable, sur toute la longueur de piste de la trajectoire, qui est inférieur au rayon de la circonférence de la surface.

2. Procédé selon la revendication 1, **caractérisé en ce que** la trajectoire se situe à au moins 90 %, de préférence à au moins 95 %, et de manière particulièrement préférable entièrement à l'intérieur de la circonférence.

3. Procédé selon l'une des revendications 1 à 2, **caractérisé en ce qu'**un rayon de courbure maximal de la trajectoire est inférieur à 99%, de préférence inférieur à 95%, et en particulier inférieur à 90% du rayon du cercle.

4. Procédé selon l'une des revendications 1 à 3, **caractérisé en ce qu'**une courbure de la trajectoire augmente de manière monotone, en particulier de manière strictement monotone, vers un centre de la circonférence.

5. Procédé selon l'une des revendications 1 à 4, **caractérisé en ce que** la trajectoire présente un point d'intersection qui est détecté au moins deux fois de manière espacée dans le temps, cela permettant en particulier de détecter un mouvement de l'œil.

6. Procédé selon la revendication 5, **caractérisé en ce que** la trajectoire présente au moins deux points d'intersection espacés l'un de l'autre et dans lequel un point d'intersection est en particulier détecté plus de deux fois de manière espacée dans le temps.

7. Procédé selon la revendication 6, **caractérisé en ce que** lesdits au moins deux points d'intersection présentent dans la projection plane un angle d'intersection qui est supérieur à 90°.

8. Procédé selon la revendication 6 ou 7, **caractérisé en ce que** le faisceau de mesure suit une trajectoire qui présente plus de deux points d'intersection, dans lequel, parmi les k*n points d'intersection, n points d'intersection se trouvent respectivement sur l'un de k anneaux concentriques.

9. Procédé selon la revendication 8, **caractérisé en ce qu'**entre les trois anneaux concentriques présentant les rayons les plus élevés, une distance entre deux anneaux concentriques adjacents diminue lorsque le rayon augmente.

10. Procédé selon la revendication 8 ou 9, **caractérisé en ce que** des points d'intersection entre la trajectoire et des anneaux concentriques alternés se trouvent respectivement sur une ligne droite orientée radialement.

11. Procédé selon l'une des revendications 1 à 10, **caractérisé en ce que** la détection des points de mesure est effectuée au moyen d'une OCT du domaine spectral ou d'une OCT à source balayée, de préférence à une fréquence constante dans le temps.

12. Procédé selon l'une des revendications 1 à 11, **caractérisé en ce que** la trajectoire est définie par des boucles, dans lequel des boucles adjacentes se chevauchent et dans lequel une boucle sur deux présente en particulier un point d'intersection qui se trouve sur un cercle concentrique à la circonférence de la surface à mesurer.

13. Procédé selon l'une des revendications 1 à 12, **caractérisé en ce que** la trajectoire est définie par deux fréquences et un rayon, en particulier par exactement deux fréquences.

14. Procédé selon la revendication 13, **caractérisé en ce que** le faisceau de mesure suit une courbe ayant pour coordonnées $(x(t) ; y(t)) = (r_0 * \sin(\omega_B t) * \cos(\omega_T t) ; r_0 * \sin(\omega_B t) * \sin(\omega_T t))$, où $r_0$ est le rayon de la circonférence de la surface à mesurer.

15. Procédé selon l'une des revendications 1 à 14, **caractérisé en ce que**, pour chaque point de mesure sur la trajectoire, un deuxième point de mesure sur la même trajectoire se trouve à une distance inférieure à 25%, de préférence inférieure à 16% du rayon.

**16.** Procédé selon l'une des revendications 1 à 15, **caractérisé en ce que**, pour chaque point à l'intérieur de la circonférence de la surface, un point de mesure se trouve sur la trajectoire à une distance d'au plus 0,5 mm, de préférence d'au plus 0,25 mm, en particulier d'au plus 0,1 mm.

**17.** Procédé d'approximation d'une section transversale d'un œil à l'aide de points de mesure détectés par un procédé selon l'une des revendications 1 à 16, dans lequel, dans la zone de la section transversale, un sous-ensemble de points de mesure détectés, qui comprennent au moins un point de mesure espacé de la section transversale, sont calculés pour obtenir une approximation de la section transversale.

**18.** Procédé selon la revendication 17, dans lequel le sous-ensemble de points de mesure détectés de deux secteurs disposés symétriquement en miroir présentent un angle au centre inférieur à 90° à l'intérieur d'une circonférence d'une surface à mesurer.

**19.** Dispositif conçu pour mettre en œuvre un procédé selon l'une des revendications 1 à 16, comprenant un équipement de mesure doté d'un système de commande permettant de guider un faisceau de mesure selon le procédé.

**Fig. 1**

**Fig. 2**

**Fig. 3**

**Fig. 4**

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1975550 A1

- EP 2417903 A

**In der Beschreibung aufgeführte Nicht-Patentliteratur**

- **RYAN P. MCNABB et al.** Distributed scanning volumetric SCOCT for motion corrected corneal biometry. *Biomedical Optics Express,* 01. September 2012, vol. 3 (9

- **VON KAROL KARNOWSKI.** *Biomedical Optics Express,* 01. September 2011, vol. 2 (9
- **S. YUN et al.** *Opt. Expr.,* 2004, vol. 12 (13), 2977